# EUROPEAN PATENT SPECIFICATION

(11) **EP 2 567 983 B1**
(45) Date of publication and mention of the grant of the patent: **13.01.2016**
(21) Application number: 11835298.8
(22) Date of filing: 29.08.2011
(51) Int. Cl.: C12N 15/62, C07K 14/705, A61K 38/17, A61P 35/00

(54) **TUMOR-TARGETED TUMOR NECROSIS FACTOR-RELATED APOPTOSIS LIGAND VARIANT AND USE THEREOF**
TUMORANZIELENDE TUMORNEKROSEFAKTOR-VERMITTELTE APOPTOSE-LIGANDEN-VARIANTE UND VERWENDUNG DAVON
VARIANT DE LIGAND D'APOPTOSE ASSOCIÉE À UN FACTEUR DE NÉCROSE TUMORALE CIBLÉ VERS UNE TUMEUR, ET SON UTILISATION

(30) Priority: 06.07.2011 CN 201110187700
(43) Date of publication of application: 13.03.2013
(73) Proprietor: Targetpharma Laboratories (Changzhou) Co., Ltd, Changzhou 213164 (CN); Changzhou Nanjing University high-tech Technology Research Institute, Changzhou 213164 (CN)
(72) Inventor: HUA, Zichun, Jiangsu Province 213164 (CN); CAO, Lin, Jiangsu Province 213164 (CN); TANG, Bo, Jiangsu Province 213164 (CN)
(74) Representative: Cabinet Laurent & Charras
(86) International application number: PCT/CN2011/001448
(87) International publication number: WO 2013/003987

(56) References cited:
- CN-A- 1 354 183
- CN-A- 1 903 373
- CN-A- 101 157 729
- US-B1- 6 284 236
- Cao Lin: "Research strategy and molecular mechanism of enhanced tumor activity of TRAIL", , 2008, XP002717430, Retrieved from the Internet: URL:http://new.med.wanfangdata.com.cn/Pape r/Detail?id=DegreePaper_Y1861505# [retrieved on 2013-12-05]
- FENG FEIXUE ET AL.: 'Relationship between neovasculature homing motif NGR and tumor targeted therapy' CHEMISTRY OF LIFE vol. 31, no. 1, February 2011, pages 27 - 30, XP008166093
- ARAP W ET AL: "CANCER TREATMENT BY TARGETED DRUG DELIVERY TO TUMOR VASCULATURE IN A MOUSE MODEL", SCIENCE, AMERICAN ASSOCIATION FOR THE ADVANCEMENT OF SCIENCE, US, vol. 279, 16 January 1998 (1998-01-16), pages 377-380, XP000857470, ISSN: 0036-8075, DOI: 10.1126/SCIENCE.279.5349.377

## Description

### Field of the Invention

The invention belongs to the field of the gene engineering technology, and refers to a preparation method and the application of a tumor-targeted TNF-related apoptosis-inducing ligand's variant.

### Background of the Invention

TNF-related apoptosis-inducing ligand (TRAIL) is one of the superfamily of the tumor necrosis factors. Similarly with others of the superfamily, soluble TNF-related apoptosis-inducing ligand is trimer, and is bound with the trimer of the acceptor molecules on the surface of the target cells to play biological action. The apoptosis-inducing action of the TNF-related apoptosis-inducing ligand is realized by transmitting death information with Death Receptors 4 (DR4) and Death Receptors 5 (DR5) in the tumor cells to each other. Although, the application of numbers of the superfamily of the tumor necrosis factors is limited because of their general toxic effect, the TNF-related apoptosis-inducing ligand is a relatively safe antitumorigenic substance which has tumor selective. In vitro, TNF-related apoptosis-inducing ligand can induce lots of tumor cells and cancer cells to apoptosis, and has preferable anti-rumor activity in the xenograft of the mouse tumor, comprising of the cancer of colon, breast carcinoma, multiple myeloma, neuroglioma, carcinoma of prostate. More importantly, TRAIL shows little or no toxicity, when administered generally to mouse and non-human primates. For the above mentioned reasons, the use of the recombinant tumor necrosis factors in treating tumor is studied in clinic.

Recently, some reports indicated that, besides of inducing rumor cells to apoptosis, also, the TNF-related apoptosis-inducing ligand relates to the natural immune and acquired immune, and the autoimmune disorders. For example, the recent research reported that it play a vital role in adjusting the negative selection and apoptosis of the thymocyte cells during the development of thymus, and inducing the autoimmune disorders, such as type I diabetes mellitus. Additionally, the receptor of the TNF-related apoptosis-reducing ligand can be expressed universally in the whole body, and the TNF-related apoptosis-reducing ligand also participates in the death of the hepatic cells and hepatitis. So it will bring unpredictable immune results if large dose of exogenous TNF-related apoptosis-inducing ligand's protein is administered repeat and generally in clinic. Because of these reports, scientists feel anxious about the potential toxic effect result from repeat and lasting administration of the TNF-related apoptosis-inducing ligand in clinic. In the potential application of the TNF-related apoptosis-inducing ligand (TRAIL) in clinic, it is a difficult problem faced to avoid the toxic effect to other tissue of the TNF-related apoptosis-inducing ligand.

### Summary of the Invention

To overcome the drawback of the wild TNF-related apoptosis-inducing ligand in the oncotherapy, the purpose of the invention is: providing a tumor-targeted TNF-related apoptosis-inducing ligand's variant, and delivering it into tumor tissues to improve the curative effect of the TNF-related apoptosis-inducing ligand and reduce its toxic effect, so that it is possible to use in the treatment of tumorous diseases.

It is verified by researches, the aminopeptidase N (APN)/CD13 protein is expressed in the endothelial cells of the new vessels of tumors (Pasqualini R, Koivunen E, Kain R etc., Cancer Res, 2000, 60:722-727). There is very little expression of the CD13 in the resting and normal endothelial cells of the vessels. Recently, the relation of the aminopeptidase N/CD13 with the tumor metastasis and prognosis has been discovered (Haraguchi N, Ishii H, Mimori K etc., J Clin Invest., 2010, 120:3326-3339; Fontijn D, Duyndam MC, van Berkel M Petc., Br J Cancer, 2006, 94:1627-1636; Fujii H, Nakajima M, Saiki I etc., Clin Exp Metastasis, 1995, 13:337-344).

To achieve the aims of the targeted delivery of the TNF-related apoptosis-inducing ligand into tumor tissues, the improvement of the curative effect, the reduction of the toxic effect, the present disclosure illustrates the following technical scheme: A tumor-targeted TNF-related apoptosis-inducing ligand's variant; it is a fusion protein of a TNF-related apoptosis-inducing ligand's variant consisting of the ligand of CD13, the connecting peptide, TNF-related apoptosis-inducing ligand by the method of the gene engineering, i.e., by artificially synthesis or recombination of the coding gene of the TNF-related apoptosis-inducing ligand's variant, soluble recombination expression and simple separation and purification, using normal method of the gene engineering. As herein disclosed, the ligand of CD13 can be a polypeptide with NGR sequence, perfectly is a polypeptide with ring structure and NGR sequence, such as short peptide of CNGRC. According to the invention, the peptide segment of the ligand of CD13 is the CNGRC peptide with a ring structure.

A short peptide of amino acid with flexible construction and non-branched chain can be added into the tumor-targeted TNF-related apoptosis-inducing ligand's variant consisting of the mentioned ligand of CD13 and the TNF-related apoptosis-inducing ligand, wherein, the short peptide has 1~25 amono-acid residues and mainly consisting of the amino without branched chain such as glycocoll, alanine, serine, etc.
When the mentioned ligand of CD13 is situated at the N-end, an amino acid, mainly alanine or glycocoll, without branched chain is added before the ligand of CD13, to avoid the degradation at the N-end during expression and to affect the function of the ligand of CD13.

The synthesized tumor-targeted TNF-related apoptosis-inducing ligand's variant which is consisted of the ligand of CD13 and TNF-related apoptosis-inducing ligand has a good application in preparation of the drugs for tumor therapy. The drugs for tumor therapy prepared by the tumor-targeted TNF-related apoptosis-inducing ligand's variant can be used in oncotherapy together with existing chemotherapy, radiotherapy, treatment by Chinese herbs, biotherapy, etc.

Furthermore, a method of soluble expression in Escherichia Coli and simple method of separation and purification for a large of high-purity polymer of the tumor-targeted TNF-related apoptosis-inducing ligand's variant. The expression of the tumor-targeted TNF-related apoptosis-inducing ligand in the Escherichia Coli is mainly inclusion body product without biological activity at present, however, the structure and the molecular of the tumor-targeted TNF-related apoptosis-inducing ligand's variant of the present invention is more complicated than the mild TNF-related apoptosis-inducing ligand, so that there will be more inclusion body generated, and the purification is more difficult. In the present invention, an expression method of the tumor-targeted TNF-related apoptosis-inducing ligand's variant with culture and induced expression at low temperature is used, so that the generation of the inclusion body in the expression production is avoid efficiently. Simultaneously, according to the biological function of the TNF-related apoptosis-inducing ligand, the present invention obtains high-purity protein production by the binding of the ion exchange chromatography and metal affinity chromatography that makes the tumor-targeted TNF-related apoptosis-inducing ligand's variant purifying efficiently. And the purity production has high percentage of polymer, so that has favorite biological activity. Wherein, the low temperature is 35°C-10°C.

The CD13 express in the endothelial cell of the tumor new vessels only, so there is very little expression in the resting normal endothelial cell of vessels. The recent research indicated that, CD13 protein is effect by basic fibroblast growth factor (bFGF) and high-selectively express in the surface of tumor cells such as 1F6 malignant mela noma, and is closely related to the malignant invasion and metastasis of tumor. We analyzed the expression level of CD13 in different cells by flow cytometer, and the results indicate that: there is high expression of CD13 in endothelial cell of human micrangium, and also high expression of CD13 in Hela tumor cell that is one of human cervical caner cells, moderate expression of CD13 in colon cancer cells HCT-15, minute quatity or no expression of CD13 molecule in colon cancer cells COLO-205. The mentioned tumor-targeted TNF-related apoptosis-inducing ligand's variant consisting of the ligand of CD13 and TNF-related apoptosis-inducing ligand can significantly improve the distribution of the TNF-related apoptosis-inducing ligand in tumor tissues, achieve the targeted delivery of the TNF-related apoptosis-inducing ligand in tumor tissues, significantly improve the anti-tumor effect of the TNF-related apoptosis-inducing ligand, significantly reduce the dose of the TNF-related apoptosis-inducing ligand simultaneously.

Simultaneously, the present invention discloses a cDNA of the tumor-targeted TNF-related apoptosis-inducing ligand's variant. It can be prepared by add coding ligand of CD13 and DNA sequence of connecting peptide to the dDNA of the TNF-related apoptosis-inducing ligand. The mentioned cDNA of the tumor-targeted TNF-related apoptosis-inducing ligand's variant can be used for gene therapy.

The tumor-targeted TNF-related apoptosis-inducing ligand's variant can be modified by the method of acidulate by polyethylene glycol and fatty acid, recombination by adding anti-body Fc fragment or x-protein, etc. to prolong the half-life of the TNF-related apoptosis-inducing ligand and obtain more favorite pharmacokinetic effect.

Compared with existing TNF-related apoptosis-inducing ligand, the present invention has the following beneficial effects:
(1) more favorite tumor-targeting characteristics: The relative selectivity of the existing TNF-related apoptosis-inducing ligand to tumor tissues mainly rely on the display of the death Receptors 4 and death Receptors 5 express in tumor tissues. However, the tumor-targeted TNF-related apoptosis-inducing ligand's variant of the present invention achieve the targeted delivery of the tumor-targeted TNF-related apoptosis-inducing ligand's variant into the tumor tissues, not only relying on the death Receptors 4 and death Receptors 5 express in tumor tissues, but also relying on the tumor characteristics of high expression of CD13.
(2) more favorite anti-tumor effect: Because of the targeted delivery among tumor tissues of the TNF-related apoptosis-inducing ligand, the tumor-targeted TNF-related apoptosis-inducing ligand's variant of the present invention, whether compared with the same dose of TNF-related apoptosis-inducing ligand, or compared with the variant RGD-L-TRAIL of integrins αVβ3,αVβ5 that is targeted to the surface of tumor cells (Chinese invention patent, application No.200710133862.1), shows more favorite anti-tumor effect where used separately or together with the existing methods of chemotharepy, radiotherapy, treatment by Chinese herbs, bioremediation.
(3) less dosage of administration: Because of the more favorite anti-tumor effect of the tumor-targeted TNF-related apoptosis-inducing ligand's variant, compared with the same dose of NF-related apoptosis-inducing ligand, when used, the dosage of administration of the protein of the tumor-targeted TNF-related apoptosis-inducing ligand's variant is significantly reduced in the case of ensuring the anti-tumor effect. The reduction of the dose of administration of the protein of the tumor-targeted TNF-related apoptosis-inducing ligand's variant can be overcome the potential toxic effect of the TNF-related apoptosis-inducing ligand when used in oncotherapy, and also can reduce the therapy cost of tumor patients, to obtain the favorite effect, low toxic effect, low cost to oncotherapy,.
(4) easy to expression and preparation: Different from the fusion protein of the tumor-targeted TNF-related apoptosis-inducing ligand targeted by tumor cell specific antibogy and the fragment thereof, the present invention combines the TNF-related apoptosis-inducing ligand and the short peptide of CD13, and the increase of molecular is limited. It is more beneficial to the gene cloning, expression and preparation of the tumor-targeted TNF-related apoptosis-inducing ligand's variant, and the yield is higher.
(5) soluble expression and simple preparation and purification: The expression of the tumor-targeted TNF-related apoptosis-inducing ligand in the Escherichia Coli is mainly inclusion body without biological activity at present, however, the structure and the molecular of the tumor-targeted TNF-related apoptosis-inducing ligand's variant of the present invention is more complex than the mild TNF-related apoptosis-inducing ligand, so that there will be more inclusion body generated, and the purification is more difficult. In the present invention, an expression method of the tumor-targeted TNF-related apoptosis-inducing ligand's variant with culture and induced expression at low temperature is used, so that the generation of the inclusion body in the expression production is avoid efficiently. Simultaneously, according to the biological function of the TNF-related apoptosis-inducing ligand, the present invention obtains high-purity protein production by the binding of the ion exchange chromatography and metal affinity chromatography that makes the tumor-targeted TNF-related apoptosis-inducing ligand's variant purifying efficiently. The present invention can obtain the purity production with high percentage of polymer by soluble expression and simple separation and purification, to resume that the product has favorite biological activity. The present invention can prepare the tumor-targeted TNF-related apoptosis-inducing ligand's variant with high percentage of polymer, which is the obvious difference from the similar studies. The present invention provides a expression method and purification process of tumor-targeted TNF-related apoptosis-inducing ligand's variant which can obtain the effective expression and high content of polymer efficiently.
(6) A amino acid without branched chain is added at the N-end of the ligand of CD13, so that the degradation of amino acid at N-end can be avoided efficiently, which will be effect the function of the ligand of CD13.

### Brief Description of the Drawings

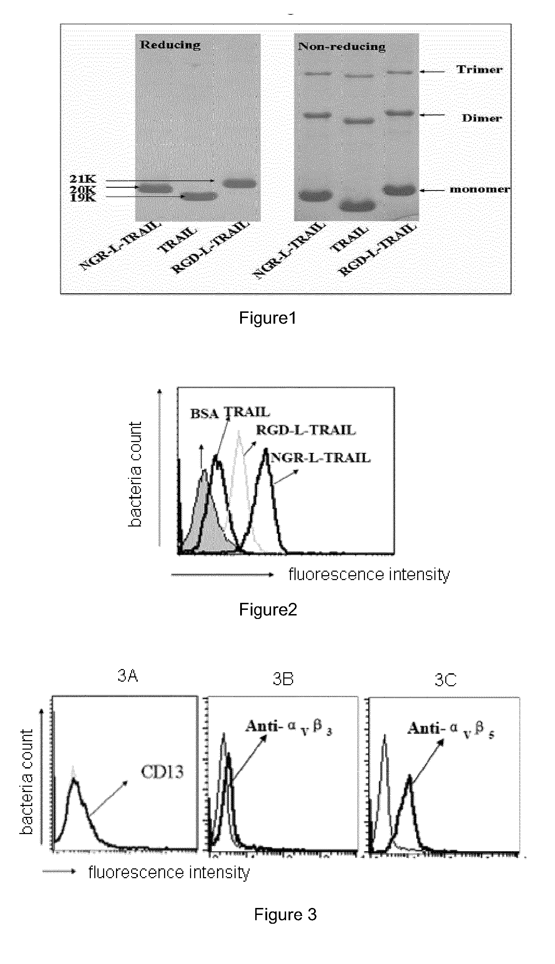

Figure 1, the analysis of the purified and recombinant human TNF-related apoptosis-reducing ligand and variant thereof.
   1A, the analytic result of SDS-PAGE: 1) tumor-targeted TNF-related apoptosis-reducing ligand's variant targeting to CD13 (NGR-L-TRAIL); 2) human TNF-related apoptosis-reducing ligand; 3) human TNF-related apoptosis-reducing ligand's variant targeting to the integrins of αVβ3 and αVβ5 (RGD-L-TRAIL).
   1B, the analytic result of non-reduced and native PAGE: 1) tumor-targeted TNF-related apoptosis- reducing ligand's variant targeting to CD13 (NGR-L-TRAIL); 2) human TNF-related apoptosis-reducing ligand (TRAIL); 3) human TNF-related apoptosis-reducing ligand's variant targeting to the integrins of αVβ3 and αVβ5 (RGD-L-TRAIL).
Figure 2, the analysis of the binding of green fluorescently labeled human TNF-related apoptosis-reducing ligand (TRAIL) and the tumor-targeted variant thereof (NGR-L-TRAIL) with human microvascular endothelial cells, by flow cytometer.
   NGR-L-TRAIL and TRAIL thereof, RGD-L-TRAIL, bovine serum albumin (BSA) as reference protein are labeled by the fluorescein. The human microvascular endothelial cells (HDMVEC) are labeled by 1 µg of labeled protein for 1 hour.
Figure 3, the analysis of the expression of the CD13 and integrins of αVβ3 and αVβ5 on the surface of COLO-205 cells.
   3A: the analysis of the expression of CD13;
   3B: the analysis of the integrin αVβ3;
   3C: the analysis of the integrin αVβ5.
Figure 4, the analysis of the dose-effect relationship of the human TNF-related apoptosis-reducing ligand (TRAIL) and the tumor-targeted variant (NGR-L-TRAIL) thereof to reduce the apoptosis if the tumor cells.
   4A: Hela cells;
   4B: CLO-205 cells;
   4C: HCT-15 cells.
Figure 5, the analysis of the effect of the human TNF-related apoptosis-reducing ligand (TRAIL) and the tumor-targeted variant (NGR-L-TRAIL) thereof to activity of the enzymes of Caspase-8 and Caspase-3 of the positive Hela cells of CD13.
   5A: Caspase-8;
   5B: Caspase-3.
   The cells is treated separately by tumor-targeted variant NGR-L-TRAI and RGD-L-TRAIL which concentration gradient is 10~270ng/ml for 8hours. After inducing, the cells are lyses on ice, and fluorogenic substrate is add to react for 1 hour, and then the analysis is carried out by microplate reader (excitation wavelength is 400nm, emission wavelength is 505nm).

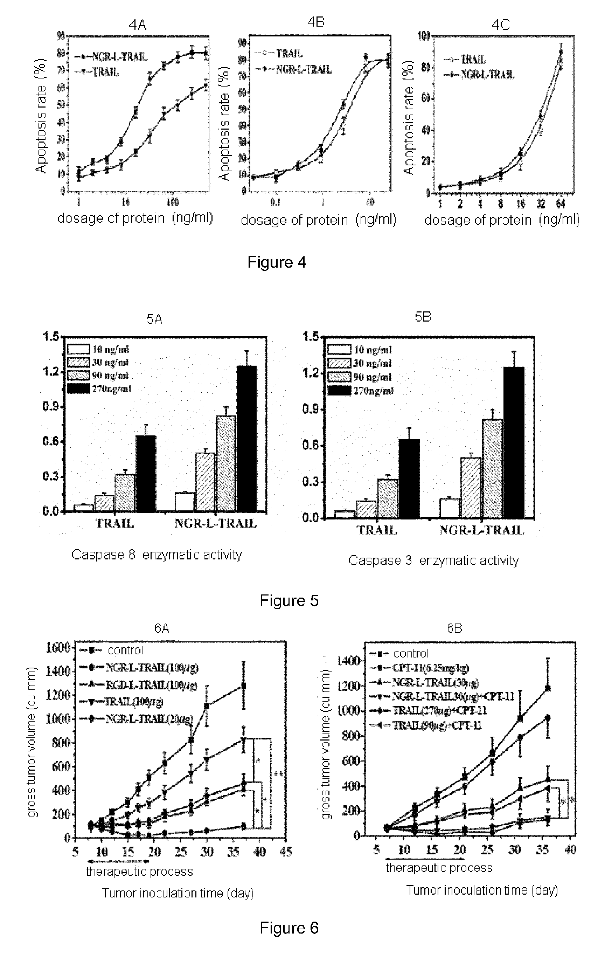

Figure 6, the efficiency against to tumor of the human TNF-related apoptosis-reducing ligand (TRAIL) and the tumor-targeted variant (NGR-L-TRAIL) monotherapy in in COLO-205 tumor model and the efficiency of that combied treatment with CPT-11 in COLO-205 tumor model.
   6A: the monotherapy of the human TNF-related apoptosis-reducing ligand (TRAIL) and the tumor-targeted variant (NGR-L-TRAIL) and RGD-L-TRIAL;
   6B: the mombined treatment of the human TNF-related apoptosis-reducing ligand and the variant thereof with CPT-11. The results of statistical analysis are showed by average numbers, wherein, the variance is standard error, the asterisk * indicates p<0.05; and two asterisks ** indicates p<0.01.

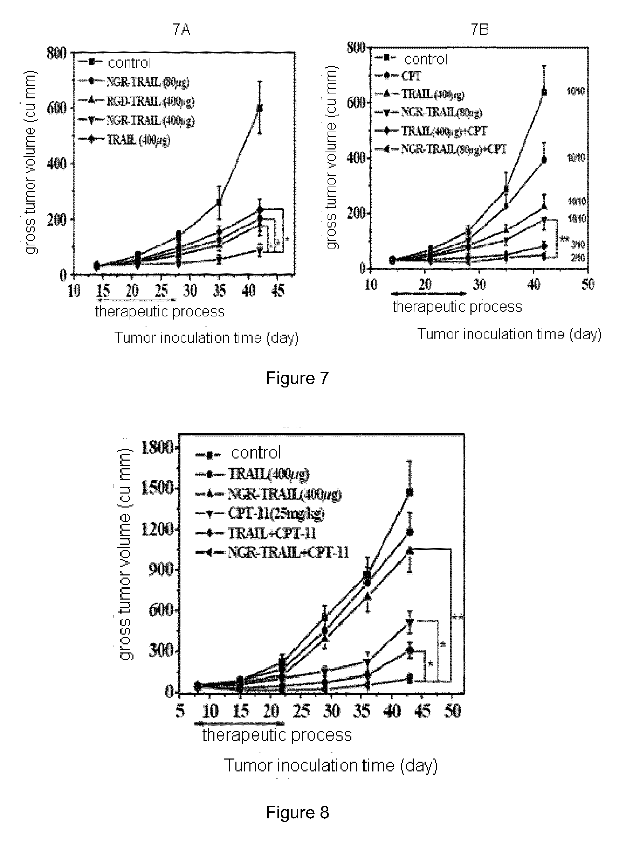

Figure 7, the efficiency against to tumor of the human TNF-related apoptosis-reducing ligand (TRAIL) and the tumor-targeted variant (NGR-L-TRAIL) monotherapy in in COLO-205 tumor model and the efficiency of that combied treatment with CPT-11 in HT-15 colon tumor model.
   7A: the monotherapy of the human TNF-related apoptosis-reducing ligand (TRAIL) and the tumor-targeted variant (NGR-L-TRAIL);
   7B: the combined treatment of the human TNF-related apoptosis-reducing ligand (TRAIL) and the tumor-targeted variant (NGR-L-TRAIL) thereof with CPT-11. The results of statistical analysis are showed by average numbers, wherein, the variance is standard error, the asterisk * indicates p<0.05; and two asterisks ** indicates p<0.01.
Figure 8, the efficiency against to tumor of the human TNF-related apoptosis-reducing ligand (TRAIL) and the tumor-targeted variant (NGR-L-TRAIL) thereof used alone and together with CPT-11 in the HT-29 colon tumor model that is insensitivity to TRAIL.

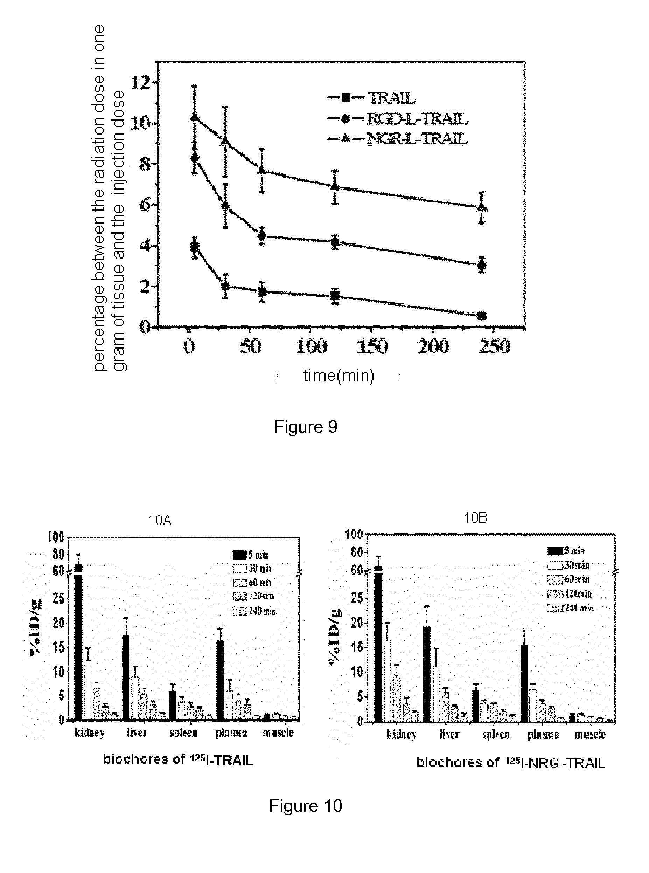

Figure 9, the comparison of the targeted enrichment effect of the human TNF-related apoptosis-reducing ligand (TRAIL) and the variant (NGR-L-TRAIL) thereof, and RGD-L-TRAIL in the tumor tissue of COLO-205 animal model of tumor.
   100µl/5mCi of the human TNF-related apoptosis-reducing ligand and the tumor-targeted variant protein thereof are administrated separately by tail intravenous injection to nude mouse with COLO-205 tumor. Tumor tissue is stripped and weighed when 5, 30, 60, 120 and 240 minutes after the injection separately. The radiation quantity of isotopes in the tumor tissue is detected by liquid scintillation counter, wherein, the unit of the radiation quantity of the tumor tissue is the percentage of the detected radiation quantity in the injection radiation quantity, based on one gram of tissue (%ID/g). All results are average values of three experiments separately.
Figure 10, the detected distribution of the human TNF-related apoptosis-reducing ligand (TRAIL) and the variant (NGR-L-TRAIL) thereof labeled by ¹²⁵I isotope in the animal tissue.

### Detailed Description of the Invention

### Embodiment 1:

The preparation of the tumor-targeted TNF-related apoptosis-inducing ligand's variant

Based on the crystal structure of the TNF-related apoptosis-inducing ligand, the short peptide of the lignad of CD13 is added to the N-end of the TNF-related apoptosis-inducing ligand, by computer aided structure modeling and molecule design, and by SGI computer workstation where the molecule design softwares (the module of Insightll, Discover etc.) of MSI company are utilized. And the amino acid length of the connecting peptide is determined by molecule modeling and molecule design.

Based on the molecule design mentioned above, firstly, we choose the design scheme where the connecting peptide has 5 glycocolls, because the computer modeling indicates that: the connecting peptide is short in this scheme, so that it will significantly affect the protein structure of the TNF-related apoptosis-inducing ligand and the binding of the CD13 and the ligand thereof. The connecting peptides in other schemes almost make a distance between the ligand of CD13 and the molecule surface of the TNF-related apoptosis-inducing ligand, so that the disturbance and the effect are light.

We also try the tumor-targeted TNF-related apoptosis-inducing ligand's variant where the alanine or glycocoll, the short peptide of alanine-glycocoll-glycocoll-serine-serine-glycocoll-glycocoll-glycocoll as connecting peptide is expressed, and the tumor-targeted TNF-related apoptosis-inducing ligand's variant with 25 amino-acid residues that contain the mentioned three repeat glycocoll-(alanine-glycocoll-glycocoll-serine-serine-glycocoll-glycocoll-glycocoll)3, and similar results are obtained. That verifies the correction of the computer molecule design. Comparatively, the expression level of the TNF-related apoptosis-inducing ligand's variant is highest (100mg/L) when 5 cysteines are added, and others are lower, but are between approximate 50-100mg/L.

During the computer aided molecule design of the tumor-targeted TNF-related apoptosis-inducing ligand's variant, based on the crystal structure of the TNF-related apoptosis-inducing ligand, we plan the molecule modeling and molecule design on the amino acid sequence and the length of the connecting peptide between the TNF-related apoptosis-inducing ligand and the ligand of CD13, by the structure modeling and molecule design of the tumor-targeted TNF-related apoptosis-inducing ligand's variant, to determine the amino acid length of the connecting peptide. The results indicate that: during computer molecule design, among the short peptide length of 1-25 amino acids, if the short peptide is consisted of the flexible amino-acid residue without branched chain, such as glycocoll, alanine, serine etc, neither the structure of the TNF-related apoptosis-inducing ligand, nor the function of the ligand of CD13 will be affected in any way. Under the short peptide length of 25 flexible amino-acid residues without branched chian, the excitation to the structure of the TNF-related apoptosis-inducing ligand is slighter when the short peptide length is smaller. Comparatively, if the length is too small, there will be some effect to the structure of the TNF-related apoptosis-inducing, and the binding of the CD13 and the lignad thereof will be affected too.

The gene of the wild human TNF-related apoptosis-inducing ligand is prepared by the reverse transcription of RNA that obtained from human placenta. The nucleotide sequences of the PCR primers of the gene of the TNF-related apoptosis-inducing ligand's variant bound CNGRC short peptide are:
Primer1: 5'-GGAATTCCATATGTGCAATGGTCGTTGCGGTGGTGGTGGTGT GAGAGAAAGAGGTCCTCAG-3';
Primer2: 5'-ATGGATCCTTAGCCA ACTAAAAAGGCCC-3'.

By PCR reaction, the gene of the tumor-targeted TNF-related apoptosis-inducing ligand's variant (NGR-L-TRAIL) with coding CNGRC short peptide and connecting peptide consisted of 5 glycocolls is obtained. The gene of the tumor-targeted TNF-related apoptosis- inducing ligand's variant (NGR-L-TRAIL) is cloned into the pET-23a expression vector of Novagen Company, and the obtained recombinant expression plasmid is expressed in Escheichia coli BL21(DE3). To obtain the soluble expression of the tumor-targeted TNF-related apoptosis- inducing ligand's variant NGR-L-TRAIL, the expression conditions are of the following: the overnight growth recombinant expression bacteria are diluted by 100 times in LB culture medium, and are cultured for 2.5 hours at 37°C, and then cultured for 1-2hours at 24°C; IPTG is added at 24°C to its concentration of 0.5mM, and then the induced expression is carried out at 24°C overnight. After the centrifugal separation, the bacteria are suspending in lysate (50mM sodium phosphate, 0.5M sodium chloride, 1mM dithiothreitol, pH 7.6) and disrupted by ultrasonic wave.

The protein of the recombinant tumor-targeted TNF-related apoptosis-inducing ligand's variant NGR-L-TRAIL is purified by supernatant passing SP-sepharose cation resin and 300mM NaCl elution peak is collected. The eluted protein is further purified by affinity chromatography with Ni-NTA agarose gel as medium, wherein the protein is eluted by 250mM imidazole and desalinated by Sephdex G-25. The water used in the experiment is super-purity water with endotoxin removed. The quantity of the protein is determined by BCA protein assay kit provided by Nanjing JianCheng Bioengineering Institute. The protein purity and molecular weight of the TNF-related apoptosis-inducing ligand and the tumor-targeted variant thereof are determined by Silver Staining of SDS-PAGE, and the molecular weight is identified by mass spectral analysis (Applied Biosystems 4700 Proteomics Analyzer).

The configuration, expression and purify of the variant RGD-L-TRAIL of integrins αVβ3,αVβ5 are executed in according with the Chinese invention patent application 200710133862.1.

In the last reports, the wild TNF-related apoptosis-inducing ligand expressed in the escherichia coli often is the form of inclusion body product without biological activity, but the tumor-targeted TNF-related apoptosis-inducing ligand's variant of the present invention, to which 4 cysteines are added (i.e. two disulfide bond), can generate inclusion body more easily when expressing in the escherichia coli, comparing with the wild TNF-related apoptosis-inducing ligand, so the purification is more difficult. By the modification of the expression condition and separation and purification, the present invention achieves the soluble expression of the tumor-targeted TNF-related apoptosis-inducing ligand's variant. And because of the purification by the cation exchange resin chromatography and Nickel metal affinity chromatography, high purity protein of the tumor-targeted TNF-related apoptosis- inducing ligand's variant with biological activity is obtained, and the yield is 100mg/L. Both the analysis (Figure 1A) of Silver Staining of 15% Polyacrylamide Gel Electrophoresis under the condition of denaturation and reduction and the sequencing analysis by Mass sepctrography identify the correction of the expression product. The ratio of the polymer in the TNF-related apoptosis-inducing ligand and the variant thereof is high (Figure 1B), that is rare in the expression and purity of the TNF-related apoptosis-inducing ligand and the similar works.

All the proteins of the superfamily of the TNF have the characteristics of forming monomer, dimer and trimer, and the biological activity of which rely on the dimer and trimer, as well as the TNF-related apoptosis-inducing ligand. To determined whether the binding of the CNGRC will affect the ability of forming polymer of the tumor-targeted TNF-related apoptosis-inducing ligand's variant, non-reduction natural polyacrylamide gel electrophoresis is carried out and the analyst result indicate that: the protein of the TNF-related apoptosis-inducing ligand and the tumor-targeted variant thereof have the ability of forming polymer. The results show that all of the TNF-related apoptosis-inducing ligand and the tumor-targeted variant thereof and RGD-TRAIL appear three strips corresponding the molecular weight of approximate 20000, 40000, and 60000 daltons. And the three strips represent the monomer, dimer and trimer separately (Figure 1B). That indicates that: the protein of the TNF-related apoptosis-inducing ligand and the tumor-targeted variant that are expressed and purified by the present invention has correct spatial structure and better biological activity than the reported TRAIL expressed in the Escherichia coli in the past.

### Embodiment 2:

The experiment of the binding of the TNF-related apoptosis-inducing ligand and endothelial cells

TNF-related apoptosis-inducing ligand and tumor-targeted variant thereof NGR-L-TRAIL, and RGD-L-TRAIL are labeled by fluorescein (Sigma Company) separately, and the nomadic fluorescein is removed from the labeled proteins by the molecular sieve Sephadex-G25. After the digestion by parenzyme, the endothelial cells of human foreskin microvascular are washed by cold phosphate buffer containing 2% of fetal bovine serum, and then are suspended again. 1µg of labeled protein is added, and incubation on ice is carried out at 4°C for 1 hour. The stained cells are washed for 3 times and analyze the binding ability by Flow Cytometer (Becton Dickinson Company) with Bovine Serum Albumin labeled by fluorescein as control.

We also evaluate the ability of the TNF-related apoptosis-inducing ligand and tumor-targeted variant thereof labeled by fluorescein, and TRAIL variant NGD-L-TRAIL (Chinese invention patent number: ZL200710133862.1) targeted to the integrins of αVβ3 αVβ5 to bind with the endothelial cells of human foreskin microvascular directly. The detect results of the Flow Cytometer indicate that: the tumor-targeted TNF-related apoptosis-inducing ligand's variant NGR-L-TRAIL has a more stronger ability to bind with the endothelial cells of human foreskin microvascular than the TRAIL variant RGD-L-TRAIL targeted to the integrins of αVβ3, αVβ5. That indicates that: the short peptide of the ligand of CD13 can improve the ability of the tumor-targeted TNF-related apoptosis-inducing ligand's variant to bind with the endothelial cells; and even the ability of the tumor-targeted TNF-related apoptosis-inducing ligand's variant to specifically bind with the endothelial cells is more stronger than the ability of the TRAIL variant RGD-L-TRAIL targeted to the integrins of αVβ3 αVβ5 to specifically bind with the endothelial cells (Figure 2). There being CD13 and integrins of αVβ3, αVβ5 during the expression in endothelial cells of human microvascular are found by different researchers, but firstly, in the present invention, the abundance of the CD13 and integrins of αVβ3, αVβ5 in the endothelial cells of human microvascular are compared and the present invention find that the expression abundance of CD13 is larger than the abundance of integrins of αVβ3 and αVβ5 significantly, and further find that CD13 is a kind of more favorite directed target molecule to the endothelial cells of human microvascular than integrins of αVβ3 and αVβ5. Integrins of αVβ3 and αVβ5 are the most recognized marker of new vessels internationally, and the aglucons containing RGD sequence are widely used as the probe for diagnosing the early growth of tumor and the early metastasis of the tumor. In present invention first proves that CD13 is a kind of more favorite directed target molecule than integrins of αVβ3 and αVβ5, and CD13 as tumor target will bring more favorite effect than integrins of αVβ3 and αVβ5. That is a result out of the expectation of the scientists of this field, and a result out of our expectation, so that one of important innovations of the present invention.

### Embodiment 3:

### The analysis of the expression of CD13 and integrins of αVβ3 and αVβ5

The expression abundance of CD13 and the integrins on the surface of cells are detected by Flow Cytomter according to the indirect labelling method. Wherein, the digested cells washed by cold phosphate buffer containing 2% of Fetal Bovine Serum, and after being suspended again, the cells are sandwiched by anti-human CD13 monoclonal antibody (eBioscience Company) or anti-human αVβ3 integrin antibody MAB23C6 (eBioscience Company) or anti-human αVβ5 integrin antibody MAB 1961 (Chemicon International Company) for 1 hour on ice, with purified isotype mouse immunoglobulin G (eBioscience Company) as negative control. After being washed twice, the cells sandwiched by primary antibody are labeled by adding secondary antibody of sheep anti mouse immunoglobulin G1 (y) (Caltag Laboratories) coupling green fluorescein for 30 minutes in the dark. The cells are washed 3 times and fixed in phosphate buffer containing 4% of formalin. The expression abundances of CD13 or the integrins of αVβ3 and αVβ5 are detected by Flow Cytometer, and all the staining experiments are repeated 3 times.

CNGRC, which is the ligand of CD13 that can form two disulfide bonds, and contain the ring structure and NGR sequence, has favorite affinity and selectivity to CD13. Because the target is designed towards to CD13 in the present invention, the expression results of CD13 on the surface of endothelial cells and tumor cells are analyzed by us, and the analysis results indicate that: there is high expression level of CD13 on the surface of human foreskin microvassular endothelial cells, and even the expression level of the CD13 on the surface of human foreskin microvassular endothelial cells is higher than the expression level of integrins of αVβ3 and αVβ5. Furthermore, It is important to note that: there are expressions of CD13 in different tumor cells in varying states, wherein, there are high expression level of CD13 in HDMVEC and Hela, intermediate abundance of expression of CD13 in colon cancer cell HCT-15, very little or no expression of CD13 in colon cancer cell COLO-205.

CD13 and integrins of αVβ3 and αVβ5 are expressed on the surface of tumor vasculars and tumor cells, however, which is a better tumor targeting target when comparing CD13 and integrins of αVβ3 and αVβ5? Nobody did detailed research and comparison about that. In the present invention, the expression levels of CD13 and integrins of αVβ3 and αVβ5 on the surface of tumor vasculars and tumor cells are be compared, and anti-tumor activity of the NGR-L-TRAIL which is the TRAIL variant targeted to CD13 and the NGR-L-TRAIL which is the TRAIL variant targeted to integrins of αVβ3 and αVβ5 is analyzed at the level of cellular level and animal model level and a surprising result that is contrary to the expectation is found. By comparison of the expression of CD13 and integrins of αVβ3 and αVβ5 on the surface of COLO-205, we find that: there very little expression of CD13 on the surface of COLO-205, but there are low expression level of integrin αVβ3 and high expression level integrin αVβ5 (Figure 3). Correspond to that, the activity of inducing COLO-205 to apoptosis of TRAIL variant NGR-L-TRAIL targeting to CD13 is increased by a little (Figure 5B), and the activity of inducing COLO-205 to apoptosis of TRAIL variant NGR-L-TRAIL targeting to integrins αVβ3 and αVβ5 is increased by 10 times (towards to COLO-205 cell, the 50% effective concentration of TRAIL is 3.5ng/ml, the 50% effective concentration of RGD-L-TRAIL is 0.37ng/ml). Hence, considering whether expression level in the target on the surfaces of COLO-205 cells, or the increase level of 50% effective concentration of NRG-L-TRAIL and RGD-L-TRAIL towards to COLO-205 cells, RGD-L-TRAIL is much superior to NGR-L-TRAIL. But in the COLO-205 tumor model experiment in vive, the anti-tumor effect of NGR-L-TRAIL is significantly superior to RGD-LTRAIL when used with the same dosage, and even the anti-tumor effect of 1/5 times dosage (20µg) of NGR-L-TRAIL is equivalent to the anti-tumor effect of 5 times dosage (100µg) of RGD-LTRAIL. The results clearly tell us that: the anti-tumor activity of the anti-tumor drugs can not be arrived by conclusion and deduction based on the activity got form the experiment in vitro and our normal logic and understanding, but should be checked gradually through scientific experiment without ostentation, and each experiment of this procedure should not be ignored. In the present invention, many experiments in vitro show that the anti-tumor activity of RGD-L-TRAIL is significantly superior to NRG-LTRAIL, but the opposite result is got from anti-tumor experiments of tumor animal model in vivo.

### Embodiment 4:

### Detecting the cell apoptosis by double staining method of annexin (Annexin V) and propidium iodide

The cells treated by the TNF-related apoptosis-inducing ligand and the tumor-targeted variant thereof NGR-L-TRAIL, and RGD-L-TRAIL are digested by trypsase and suctioned off culture plate, and after being washed twice by phosphate buffer, the cells are centrifugalize by 300g of centrifugal pull for 5minutes. The supernatant is removed and the cells are suspended again by 100µl binding buffer. Annexin V-FITC (BD Pharmgen Company) is added to which final concentration is 2µg/ml, and the cells are incubated at the room temperature. 10 minutes later, 400µl binding buffer is supplemented. The cells are transferred into Flow Analyzer Tube, and 1µg propidium iodide (Sigma Company) is added into each of the tube. The cells are analyzed by Flow Cytometer within 30minutes. The experiment is repeated three times for each cell line.

The activity to induce the tumor cell apoptosis of the tumor-targeted TNF-related apoptosis-inducing ligand's variant NGR-TRAIL is appraised using COLO-205, HCT-15 and HT-29 cells separately (Figure 4). After induced by series concentration gradient TRAIL variant or TRAIL, the tumor cells are detected by Flow Cytometer according to the double staining method of Annexin V-FITC and PI. The result shows that: the sensibility of different tumor cells to TRIAL is diversity, wherein, COLI-205 cells are most sensitive, HCT-15 cells are the secondary, Hela cells are not sensitive relatively. However, in Hela cells, the activity to induce tumor cells apoptosis of the NGR-L-TRAIL is increased significantly. The 50% effective concentration (EC50) of NGR-L-TRIAL to Hela cell is approximate 18.5ng/ml, the 50% effective concentration (EC50) of TRAIL to Hela cell is approximate 145ng/ml. The sensibility of Hela cell to TRAIL is increased 8 times because of the addition of the short peptide. To COLO-205 and HCT-15, the activity to induce tumor cells apoptosis of NGR-L-TRAIL is higher than the one of TRAIL a little. There is a positive correction between the difference of the apoptosis-inducing to these tumor cells between NGR-L-TRAIL and TRAIL and the expression and the abundance of the CD13 molecule on the surface of tumor cell. In the Hela tumor cells in which the expression level of CD13 is high, the activity to induce the tumor cells apoptosis of NGR-L-TRAIL is increased significantly. The NGR domain from NGR-L-TRAIL can direct the protein of TRAIL variant to the surface of target cell to enrichment on the cell surface. The result from that is the increasing of the local concentration of tumor-targeted TNF-related apoptosis-inducing ligand's variant, thus the TNF-related apoptosis-inducing ligand that is a part of the variant molecule can be closed to TRAIL receptor easily, frequently and efficiently so that the signal to active the pathways of apoptosis is enhanced and the activity of NGR-L-TRAIL is increased. And also, the enhance of increase of the activity to induce the apoptosis relies on the abundance of the expression of CD13 on the surface of the tumor cells, wherein, the higher abundance, the higher activity, and vice versa. For example, in the Hela tumor cells expressing CD13 highly, because of the overexpression of CD13, the activity of NGR-L-TRAIL is increased by 8 times (Figure 4). Contrary, the activity of NGR-L-TRAIL is increased little in COLO-205 colon cancer cells expressed CD13 lowly. The results above clearly indicate that: the increase of the activity of tumor-targeted TNF-related apoptosis-inducing ligand's variant results from the targeting of CNGRC.

### Embodiment 5:

### Detecting the enzyme activities of Caspas-8 and Caspas-3

The enzyme activities of Caspas-8 and Caspas-3 are detected by Fluorometric Assay Kit (Oncogene Company) according to the experiment method provide by the company. The fluorescence value is detected by Microplate Reader, wherein, the fluorescent parameters are: 400nm of excitation wavelength and 505nm of emission wavelength.

Different from tumor cells, even the normal endothelial cells of human foreskin, 293T kidney cells and primary-cultured hepatocytes are treated by the tumor-targeted TNF-related apoptosis-inducing ligand's variant which concentration is 300ng/ml for 24hours, there is no significant cytotoxicity being found. The result shows that the tumor-targeted TNF-related apoptosis-inducing ligand's variant can distinguish the normal cells from tumor cells, and induce tumor cells to apoptosis, however is safe to normal cells.

We detected the activity of Caspase-8 and Caspase-3 in Hela cells that are treated by TNF-related apoptosis-inducing ligand and the tumor-targeted variant thereof, by fluorometry. Campared with the same dosage of TRAIL, NGR-L-TRAIL shows higher activity of Caspase-8 and Caspase-3 (Figure 5A, 5B). It indicates that, because NGR main of NGR-L-TRAIL can direct TRAIL variant protein onto the surface of the targeting cells to enrichment on the surface of the cells, the local concentration of tumor-targeted TNF-related apoptosis-inducing ligand's variant increases. Thus, the TNF-related apoptosis-inducing ligand that is a part of variant molecule can more easily, frequently and efficiently close to TRAIL acceptor, to increase the signal to active the pathways of apoptosis. In the mutated Jurkat cells from FADD^{-/-} and Caspase-8^{-/-}, the inducing apoptosis cannot be detected when TNF-related apoptosis-inducing ligand and the variant thereof used. It indicates that, TNF-related apoptosis-inducing ligand, as well as the variant thereof, plays a role in inducing apoptosis by means of acceptor-FADD-Caspase-8.

### Embodiment 6:

### The experiment of anti-tumor effect in tumor animal model

The female nude mice bought from ShangHai Experiment Animal Center are 5-6 weeks old. The mouse tail intravenous injection of 100µg of purified Asialo GM-1 antibody (Wako Chemicals Company, Japan) that is specific antibody blocking natural killer cells is carried out first. 24 hours later, 100 thousands of COLO-205, HT-15 and HT-29 colon cancer cells is subcutaneously vaccinated at the top right side of the back of a mouse. When the volume of tumor arrives 70 cubic millimeters, the mice are grouped randomly and treated. The recombinant TNF-related apoptosis-inducing ligand and its targeted variant NGR-TRAIL, and the protein of RGD-L-TRAIL variant are intraperitoneally injected once a day continued for 10 to 14 days. Hydrosoluble camptothecin CPT-11 (11-hydroxyl-camptothecin, trade name: Campto, from Pharmacia/Upjohn Company) is administrated by intravenous administration once a week, for twice in all. The recombinant protein and camptothecin are diluted by phosphate buffer. The volume of tumor is detected by vernier caliper and calculated according the formula: length is multiplied by the square of width and then is divided by 2.

Two colon cancer models of COLO-205 and HT-15 are utilized to detect and compare the anti-tumor activity of TNF-related apoptosis-inducing ligand and its tumor-targeted variant NGR-L-TRAIL in athymic nude mice. Because of the sensitivity of COLO-205 and HCT-15 colon cancer cells to TRAIL (wherein COLO-205 is more sensitive), we assessed the treatment effect of NGR-L-TRAIL and TRAIL separately in two models. As shown in Figures 4 and 6, NGR-L-TRAIL inhibited the tumor growth significantly and the growth inhibitory activity to tumor of it is higher than that of the wild TRAIL and RGD-L-TRAIL.

In the COLO-205 model, the growth inhibitory activity to tumor of 100µg/day dosage of NGR-L-TRAIL is far better than 100µg/day dosage of wild TRAIL (p<0.01); and the growth inhibitory activity to tumor of 100µg/day dosage of NGR-L-TRAIL is far better than 100µg/day dosage of RGD-L-TRAIL (p<0.01) (p<0.05), and even the dosage of NGR-L-TRAIL is cut down to the 1/5 times (20µg/day) of the wild's, the growth inhibitory activity to tumor is better than the one of the wild TRAIL (100µg/day) (p<0.05); and the growth inhibitory activity to tumor of 20µg/day dosage of NGR-L-TRAIL is nearly to the growth inhibitory activity to tumor of 100µg/day dosage of RGD-L-TRAIL (Figure 6).

In the HCT-15 model, the growth inhibitory activity to tumor of NGR-L-TRAIL is better than wild TRAIL (p<0.01), and better than RGD-L-TRAIL too(p<0.05), at the same dosage (400µg/day); and, when its dosage is cut down to the 1/5 times (80µg/day) of the dosage of wild TRAIL, its growth inhibitory activity to tumor is equal to the one of the wild TRAIL (400µg/day) (there is no significant difference between them) (Figure 7). The growth inhibitory activity to tumor of NGR-L-TRAIL of the dosage of 80µg/day is nearly to the growth inhibitory activity to tumor of RGD-L-TRAIL of the dosage of 400µg/day. During the treatment, the growth inhibitory activity to tumor of NGR-L-TRAIL relies on the dosage.

The results above show that: the combination of CNGRC with TRAIL significantly increases the anti-tumor activity of TRAIL, in vivo; furthermore, anti-tumor effect of NGR-L-TRAIL significantly exceeds the one of RGD-L-TRAIL.

The anti-tumor effect of tumor-targeted TRAIL used together with chemotherapeutic drug CPT-11 is researched in the present invention. Through COLO-205, HC-15 and HT-29 models of athymic nude mice, the anti-tumor effect of NGR-L-TRAIL used together with chemotherapeutic drug CPT-11 is researched, wherein, COLO-205, HC-15 are sensitive to TRAIL, and COLO-205 is most sensitive, and HT-29 is insensitive to TRAIL. The protein of NGR-L-TRAIL or TAIL is intraperitoneally injected once a day for two weeks, and CPT-11 is tail intravenously injected once every two days up to 7 injections. In the group of combining administration, for COLO-205 model that is sensitive to TRAIL, a less dosage of CPT-11 (6.25mg/kg per time) is administrated with different dosage of NGR-L-TRAIL (30 or 80µg/day per mouse) or TRAIL (270µg/day per mouse or 90µg/day per mouse); for HT-29 colon cancer model that is insensitive to TRAIL, a larger dosage of CPT (25mg/kg per time) is administrated with NGR-L-TRAIL or TRAIL (400µg/day per mouse).

In COLO-205 model, the growth inhibitory activity to tumor of the group in which NGR-L-TRAIL which dosage is 30µg/day is administrated combining with CPT-11 which dosage is 6.25mg/kg per time is stronger than that of the group in which NGR-L-TRAIL is administrated lonely with the dosage of 30µg/day (p<0.05); is equal to that of the group in which TRAIL which dosage is 270µg/day is administrated combining with CPT-11 which dosage is 6.25mg/kg per time; and is stronger than that of the group in which TRAIL which dosage is 90µg/day is administrated combining with CPT-11 which dosage is 6.25mg/kg per time (p<0.05) (Figure 6).

In HCT-15 model, the growth inhibitory activity to tumor of the group in which NGR-L-TRAIL which dosage is 80µg/day is administrated combining with CPT-11 which dosage is 6.25mg/kg per time is stronger than that of the group in which CPT-11 which dosage is 6.25mg/kg per time or NGR-L-TRAIL which dosage is 80µg/day is administrated lonely; and is stronger than that of the group in which TRAIL which dosage is 400µg/day per mouse is administrated combining with CPT-11 which dosage is 6.25mg/kg per time (Figure 7). It is worthy mentioning that, in the group in which NGR-L-TRAIL which dosage is 400µg/day per mouse is administrated combining with CPT-11, at the 28^{th} day, there are 8 tumor disappeared mice from the 10 administrated mice; in the group in which TRAIL which dosage is 400µg/day per mouse is administrated combining with CPT-11, at the 28^{th} day, there are 7 tumor disappeared mice from the 10 administrated mice (Figure 7B).

In HT-29 colon cancer model insensitive to TRAIL, when NGR-L-TRAIL is used lonely, even the intraperitoneal injection dosage is up to 400µg/day per mouse, only weak anti-tumor effect can be found. However, when NGR-L-TRAIL combines with CPT-11 to treatment, the growth of tumor is inhibited significantly, and the effect is better than that of combination of TRAIL with CPT-11. When NGR-L-TRAIL which dosage is 400µg/day combining with CPT-11 which dosage is 25mg/kg/day, the growth inhibitory activity to tumor is best, and far better than the group in which NGR-L-TRAIL which 400µg/day is administrated (p<0.01), and better than that of the group in which CPT-11 which dosage is 25mg/kg/day (p<0.05), and also, is better than that of the group in which TRAIL which dosage is 400µg/day is administrated combining CPT-11 which dosage is 25mg/kg/day (p<0.05).

Generally speaking, in the above mentioned cancer models, when combing with CPT-11, the anti-tumor activity of NGR-L-TRAIL is stronger than the effect when using NGR-L-TRAIL or CPT-11 lonely, and the effect of NGR-L-TRAIL combing with CPT-11 is better than the effect of TRAIL combining with CPT-11. When the dosage of NGR-L-TRAIL is 1/9~1/3 and 1/5 dosage of TRAIL separately, which inhabitation effect to COLO-205 and HCT-15 tumor that is sensitive is equal to the effect of TRAIL. When NGR-L-TRAIL combines with CPT-11, which growth inhibitory activity to HT-29 tumor that is insensitive to TRAIL is better than that of same dosage of TRAIL combining CPT-11. The results show that: when combining with CPT-11, the anti-tumor activity of the tumor-targeted variant protein NGR-L-TRAIL is significantly improved compared with which using lonely, and the growth inhibitory activity to tumor is better than the combination of wild TRAIL with chemotherapeutic drugs. Furthermore, the combination expands the scope of its application. The tumor- targeted variant protein can efficiently inhibit the growth of tumors that is insensitive to TRAIL (such as HT-29 colon cancer).

The animal model experiments in vivo prove the better effect of the tumor-targeted TNF-related apoptosis-inducing ligand's variant NGR-L-TRAIL than wild TNF-related apoptosis-inducing ligand, and the variant RGF-L-TRAIL of TRAIL targeted intergrin αVβ3 and intergrin αVβ5. It indicates that, when combining with tumor-targeted peptide targeted CD13, the TNF-related apoptosis-inducing ligand can increase the anti-tumor biological activity at the level of animal tumor model. Similarly, the TNF-related apoptosis-inducing ligand's variant not only increase the effect when used lonely, but also has more significant effect when combining with chemotherapeutic drugs because the significant synergistic effect. The combination of the TNF-related apoptosis- inducing ligand's variant with CPT-11 not only can decrease the their dosage, minimize the potential systemic toxicity, but also can expand the scope of application to the tumor that is insensitive to TNF-related apoptosis-inducing ligand originally (Figure 8). The animal experiments in vivo prove that the treatment effect of the tumor-targeted TNF-related apoptosis-inducing ligand's variant is favorite than the TNF-related apoptosis-inducing ligand, even than the variant RGF-L-TRAIL of TRAIL targeted intergrin αVβ3 and intergrin αVβ5.

### Embodiment 7:

### The test of drug distribution of recombinant protein in tumor tissue

The recombinant TNF-related apoptosis-inducing ligand's variant NGR-L-TRAIL, and variant RGD-L-TRAIL is separately labeled by the kit labeled by the radioisotope ¹²⁵I . The results of the labeling experiment are that: the specific activity of ¹²⁵I-TNF-related apoptosis-inducing ligand is 7.86µCi per one µg of protein, and the specific activity of ¹²⁵I-TNF-related apoptosis-inducing ligand's variant is 7.49µCi per one µg of protein. After implanting the COLO-205 colon tumor, the nude mice are randomized for mild group and two variant groups when the tumor volume is up to 400 to 500 cubic millimeters. 5 time points, that are 5, 30, 60, 120 and 240minutes separately, are set. At each time point 3 animals are selected, and 5µCi of labeled protein is injected for each tumor-bearing nude mouse. At each time point, the tumor tissue is enulcleated by surgical operation from the mouse and weighed. The radiological dose is detected by Liquid Scintillation Counter. The abundance of labeled protein in tumor tissue is calculated by the percent of the detected radiological dose per gram of the tissue to injection radiological dose (%ID/g).

To further prove that the increase of the anti-tumor activity of the TNF-related apoptosis-inducing ligand's variant in animal model results from its enrichment into tumor tissue, and to compare the targeting capacity of NGR-L-TRAIL and RGD-L-TRAIL in tumor animal model, the distributions of the TNF-related apoptosis-inducing ligand's variant NGR-L-TRAIL and RGD-L-TRAIL labeled by isotope ¹²⁵I, and mild TRAIL in tumor tissue are detected. The TNF-related apoptosis-inducing ligand and the variant thereof labeled by the same radiological dose ¹²⁵I are injected in to COLO-205 tumor-bearing nude mice separately. 5, 30, 60, 120 minutes after the injection, tumor tissues of the mice are enulcleated by surgical operation and weighed. The radiological doses of the isotope are detected by Liquid Scintillation Counter separately. The results show that: when the TNF-related apoptosis-inducing ligand TRAIL combines with the short peptide ligand CNGRC of CD13, and the short peptide ligand ACDCRGDCFC of intergrin αVβ3 and intergrin αVβ5, the enrichment capacity of TRAIL protein target into COLO-205 tumor tissues is increased significantly and NGR-L-TRAIL can be exist and specifically enriched in tumor tissues. Just after the injection, the abundance of ¹²⁵I-RGD-L-TRAIL is approximately 2 times as much as that of ¹²⁵I-TRAIL in tumor tissue, and the abundance of ¹²⁵I-NGR-L-TRAIL is approximately 2.5 times as much as that of ¹²⁵I-TRAIL in tumor tissue, and the abundance of ¹²⁵I-NGR-L-TRAIL in COLO-205 tumor tissue is significantly higher than that of ¹²⁵I-TRAIL (Figure 9). Because of the increase of the affinity of NGR-L-TRAIL and RGD-L-TRAIL to tumor tissues and the expanded distribution in tumor tissues, their rate to be removed in circulatory blood is slowed down greatly, so that their distributing time is prolonged. 240 minutes after the injection, it is difficult to find the distribution of TRAIL in tumor tissue, but there still is quite a few of RGD-L-TRAIL distributing in the tumor region (Figure 9), and the content of NGR-L-TRAIL in tumor tissues is twice as much as the content of RGD-L-TRAIL. The above results show that, when combining with TRAIL, the peptide CNGRC retains the biological function of the peptide CNGRC to be bound to vascular endothelial cells, and of inhibition its hyperplasia and of the inducing apoptosis. Thus, both the facts of the increase of the distribution in tumor tissues and of the anti-tumor activity detected in animal models of TNF-related apoptosis-inducing ligand's variant prove that the TNF-related apoptosis-inducing ligand's variant NGR-L-TRAIL provided by the present invention can give the TNF-related apoptosis-inducing ligand better and stronger tumor targeting captivity than TGD-L-TRAIL, and can decrease the dose of administration, and finally can improve the anti-tumor effect. It is the first to improve that the ligand of CD13, which targeting captivity is favorite than RGD ligands of intergrins that is used for early diagnosis of tumor, is a favorite probe for the diagnosis of tumor in the present invention.

### Embodiment 8:

### Binding of the recombinant protein and tumor tissue in vivo

TNF-related apoptosis-inducing ligand and the tumor-targeted variant NGR-L-TRAIL, RGD-L-TRAIL, Bovine Serum Albumin are labeled by green fluorescein. The nomadic fluorescein is removed by gel molecular sieve Sephadex-G25 from the labeled protein. 500µg of protein labeled by green fluorescein is injected into the tumor-bearing nude mouse when the volume of tumor tissue is up to 400 to 500 cubic millimeters by tail vein injection. 30 minutes after the injection, tumor tissue of the mouse is enulcleated by surgical operation to prepare the single-cell suspension of the tumor cells. After washed by physiological saline for several times, 60 thousands of cells are selected to be detected by Flow Cytometer to analyze the binding of the recombinant protein to the surface of the tumor cells.

The statistical analysis of the data is carried out by the software of Statistical Package for the Social Science. All of the experiments are repeated for at least 3 times. The results of apoptosis-inducing and adhesion experiments are stood for by average standard deviation, and the volume of tumor is stood for by standard error. When p is less than 0.05, the significance difference is deemed to; and when p is less 0.01, the extremely significance difference is deemed to, marked by one or two asterisks separately.

The distribution of NGR-L-TRAIL in other tissues is similar to TRAIL, and there is no specificity enrichment in other visceral organs (Figure 10A, Figure 10B). The metabolite of NGR-L-TRAIL in vivo is eliminated from the body by kidney chiefly (Figure 10B).

Thus, the increase of the distribution in the tumor region in the body of animals and the increase of the anti-tumor activity that is detected in animal models fully demonstrate that NGR-L-TRAIL designed by our molecular design is superior to RGD-L-TRAIL, and can give favorite targeting captivity to TRAIL protein and finally can increase the anti-tumor effect.

## Claims

1. A variant of a tumor-targeted TNF-related apoptosis-inducing ligand (TRAIL) which is a fusion protein consisting of a peptide segment of a ligand of CD13, a connecting peptide, and the TNF-related apoptosis-inducing ligand (TRAIL) wherein the peptide segment of the ligand of CD13 is the CNGRC peptide with a ring structure.

2. A TRAIL variant according to Claim 1 wherein the number of the amino-acid residue in the connecting peptide is between 1 and 25.

3. A TRAIL variant according to any of Claims 1 to 2 wherein it is modified by an acidulation with polyethylene glycol and fatty acid, and by a recombination to add the Fc fragment of an antibody or albumin.

4. A method of preparing a TRAIL variant according to any of Claims 1 to 3 comprising:
the gene engineering expression and separation and purification carrying out the routine gene engineering method of the coding gene of a tumor targeted TNF related apoptosis inducing ligand's variant structured by artificial synthesis or gene cloning.

5. A method for the soluble expression of the TRAIL variant according to any of Claims 1 to 3 in *Escherichia coli*, wherein the recombinant bacterial strain of *Escherichia coli* expressing the TRAIL variant according to any of Claims 1 to 3 is cultured and induced to the expression at low temperature, wherein the low temperature is 35°C-10°C.

6. A method for separating and purifying the TRAIL variant wherein the TRAIL variant according to any of Claims 1 to 3 is purified by ion exchange chromatography and metal affinity chromatography.

7. A cDNA which encodes the TRAIL variant of any of Claims 1 to 3, wherein it comprises continuous sequences encoding a CD13 ligand and a connecting peptide.

8. A gene therapy vector comprising the cDNA according to Claim 7.

9. The TRAIL variant according to any of Claims 1 to 3 for use in tumor therapy.

10. The TRAIL variant according to any of Claims 1 to 3 for use in tumor therapy in combination with the existing drugs and technologies for tumor therapy.

11. The cDNA according to Claim 7 or the gene therapy vector according to claim 8 for use in tumor therapy.

12. The cDNA according to Claim 7 or the gene therapy vector according to claim 8 for use in tumor therapy in combination with the existing drugs and technologies for tumor therapy.

## Patentansprüche

1. Variante eines tumoranzielenden, TNF-vermittelten (Tumornekrosefaktorvermittelten), Apoptose einleitenden Liganden (TRAIL), welcher ein Fusionsprotein ist, bestehend aus einem Peptidsegment eines Liganden für CD13, einem Verbindungspeptid und dem TNF-vermittelten, Apoptose einleitenden Liganden (TRAIL), wobei das Peptidsegment des Liganden für CD13 das CNGRC-Peptid mit einer Ringstruktur ist.

2. TRAIL-Variante nach Anspruch 1, wobei die Anzahl der Aminosäurerückstände im Verbindungspeptid zwischen 1 und 25 liegt.

3. TRAIL-Variante nach einem der Ansprüche 1 bis 2, wobei sie durch eine Ansäuerung mit Polyethylenglykol und Fettsäure und durch eine Rekombination modifiziert ist, um das Fragment Fc eines Abwehrstoffs oder Albumins beizumischen.

4. Verfahren zur Bereitstellung einer TRAIL-Variante nach einem der Ansprüche 1 bis 3, umfassend:
die gentechnische Expression und Separation und Purifikation, die das übliche gentechnische Verfahren des codierenden Gens einer tumoranzielenden, TNF-vermittelten, Apoptose einleitenden Ligandenvariante durchführt, strukturiert durch künstliche Synthese oder Genklonierung.

5. Verfahren für die lösliche Expression der TRAIL-Variante nach einem der Ansprüche 1 bis 3 in *Escherichia coli,* wobei der die TRAIL-Variante nach Anspruch 1 bis 3 darstellende, rekombinierte Bakterienstamm von *Escherichia coli* gezüchtet und bei niedriger Temperatur zu der Expression angeregt wird, wobei die niedrige Temperatur 35°C - 10°C beträgt.

6. Verfahren zur Entmischung und Reinigung der TRAIL-Variante, wobei die TRAIL-Variante nach einem der Ansprüche 1 bis 3 durch Ionenaustauschchromatographie und Metallaffinitätschromatographie gereinigt wird.

7. Eine cDNS (komplementäre DNS), welche die TRAIL-Variante nach einem der Ansprüche 1 bis 3 codiert, wobei sie kontinuierliche Sequenzen enthält, die einen Liganden für CD13 und ein Verbindungspeptid codieren.

8. Gentherapievektor, umfassend die cDNS nach Anspruch 7.

9. TRIAL-Variante nach einem der Ansprüche 1 bis 3 zur Verwendung bei einer Tumortherapie.

10. TRIAL-Variante nach einem der Ansprüche 1 bis 3 zur Verwendung bei der Tumortherapie in Kombination mit den bestehenden Medikamenten und Techniken für Tumortherapie.

11. Die cDNS nach Anspruch 7 oder der Gentherapievektor nach Anspruch 8 zur Verwendung bei einer Tumortherapie.

12. Die cDNS nach Anspruch 7 oder der Gentherapievektor nach Anspruch 8 zur Verwendung bei einer Tumortherapie in Kombination mit den bestehenden Medikamenten und Techniken für Tumortherapie.

## Revendications

1. Un variant d'un ligand induisant une apoptose liée au TNF ciblant une tumeur (TRAIL), qui est une protéine de fusion constituée d'un segment peptidique d'un ligand de CD 13, un peptide de liaison, et le ligand induisant l'apoptose liée au TNF (TRAIL), **caractérisé en ce que** le segment peptidique du ligand CD13 est le peptide CNGRC avec une structure cyclique.

2. Un variant TRAIL selon la revendication 1, **caractérisé en ce que** le nombre des résidus d'acides aminés dans le peptide de liaison est comprise entre 1 et 25.

3. Un variant TRAIL selon l'une quelconque des revendications 1 à 2, **caractérisé en ce qu'**il est modifié par une acidification avec du polyethylene glycol et d'acide gras, et par une recombinaison pour ajouter le fragment Fc d'un anticorps ou l'albumine.

4. Procédé de préparation d'un variant TRAIL selon l'une quelconque des revendications 1 à 3, comprenant: la construction génétique, l'expression et la séparation et la purification, mettant en oeuvre des méthodes de génie génétique usuelles, du gène codant un variant d'un ligand induisant une apoptose liée au TNF ciblant une tumeur, structuré par synthèse artificielle ou de clonage de gènes.

5. Procédé pour l'expression soluble d'un variant TRAIL selon l'une quelconque des revendications 1 à 3 dans *Escherichia coli*, **caractérisé en ce que** la souche bactérienne recombinante d'*Escherichia coli* exprimant le variant TRAIL selon l'une quelconque des revendications 1 à 3 est cultivée et son expression est induite à basse température, dans lequel la basse température est de 35 °C-10 °C.

6. Procédé pour séparer et purifier le variant TRAIL, **caractérisé en ce que** le variant TRAIL selon l'une quelconque des revendications 1 à 3 est purifié par chromatographie d'échange d'ions et chromatographie d'affinité métallique.

7. Un ADNc qui code pour le variant TRAIL de l'une quelconque des revendications 1 à 3, **caractérisé en ce qu'**il comprend des séquences continues codant pour un ligand de CD 13 et un peptide de liaison.

8. Un vecteur de thérapie génique comprenant l'ADNc selon la revendication 7.

9. Le variant TRAIL selon l'une quelconque des revendications 1 à 3, pour son utilisation en thérapie tumorale.

10. Le variant TRAIL selon l'une quelconque des revendications 1 à 3 pour son utilisation en thérapie tumorale en combinaison avec des médicaments et des technologies pour le traitement des tumeurs existants.

11. L'ADNc selon la revendication 7 ou le vecteur de thérapie génique selon la revendication 8 pour son utilisation en thérapie tumorale.

12. L'ADNc selon la revendication 7 ou le vecteur de thérapie génique selon la revendication 8 pour son utilisation en thérapie tumorale en combinaison avec des médicaments et des technologies pour le traitement des tumeurs existants.
